# EUROPÄISCHE PATENTSCHRIFT

(11) **EP 3 281 587 B1**
(45) Veröffentlichungstag und Bekanntmachung des Hinweises auf die Patenterteilung: **25.12.2019**
(21) Anmeldenummer: 17183284.3
(22) Anmeldetag: 26.07.2017
(51) Int. Cl.: A61B 17/135, A61B 17/00

(54) **BLUTSPERREMANSCHETTE**
TOURNIQUET CUFF
MANCHON DE GARROTAGE

(30) Priorität: 12.08.2016 DE 102016115027
(43) Veröffentlichungstag der Anmeldung: 14.02.2018
(73) Patentinhaber: ulrich GmbH & Co. KG, 89081 Ulm (DE)
(72) Erfinder: Erdmann, Sven, 89081 Ulm (DE); Striggow, Uwe, 72631 Aichtal (DE); Wimböck, Sarah Andrea Diana, 89075 Ulm (DE)
(74) Vertreter: Charrier Rapp & Liebau

(56) Entgegenhaltungen:
- BE-A- 459 897
- DE-A1- 3 333 311
- US-A- 2 031 870
- US-A- 2 045 750

## Beschreibung

Die Erfindung betrifft eine Blutsperremanschette zum Anlegen an ein Körperglied und zur Erzeugung einer Blutsperre in dem Körperglied, nach dem Oberbegriff des Anspruchs 1 sowie ein Verfahren zur Herstellung einer solchen Blutsperremanschette.

Aus dem Stand der Technik, z.B. der US 4637394-B, sind Blutsperreeinrichtungen in Form von aufblasbaren Manschetten bekannt, welche an einem Körperglied angelegt und zur Erzeugung einer Blutsperre in dem Körperglied aufgeblasen werden können. Die aufblasbare Manschette wird zur Erzeugung einer Blutsperre in dem Körperglied mit einer Druckgasquelle verbunden und mit einem Druckgas (bspw. Druckluft) soweit aufgeblasen, dass die Manschette auf das Körperglied einen Druck ausübt, der groß genug ist, um den Blutdurchfluss in dem von der Manschette umschlossenen Abschnitt des Körperglieds zumindest weitgehend oder vollständig zu unterbrechen. Derartige Blutsperreeinrichtungen werden beispielsweise bei Operationen an Körpergliedern eingesetzt, bei denen eine weitgehende oder vollständige Blutsperre oder sogar eine Blutleere im Operationsbereich bereitgestellt und während der Operation aufrecht erhalten werden soll, um die Operation zu vereinfachen oder das Risiko von Verletzungen des Gefäßsystems zu reduzieren. Zum Aufblasen der Manschette wird diese über eine Leitung mit einer regelbaren Druckgasquelle verbunden, mit der die Manschette mit einem variablen, vorgebbaren Druck aufgeblasen werden kann. Die Druckgasquelle wird dabei von einer Steuereinrichtung angesteuert, mit welcher ein Bediener das Aufblasen der Manschette und auch das Ablassen des Drucks zur Beendigung der Blutsperre steuern kann.

Aus der DE 33 33 311 A1 ist eine Blutsperremanschette mit einer äußeren Manschettenhülle aus einer Innenwandung und einer Außenwandung, die jeweils aus einem Gewebe bestehen, und einem in der Manschettenhülle angeordneten Blähkörper in Form einer Gummiblase bekannt.

Aus der US 2 045 750 A ist eine Blutsperremanschette mit einem Trägerkörper aus Metall oder Gummi mit einem daran befestigten Blähkörper bekannt. Der Blähkörper ist dabei nicht in Längsrichtung des Trägerkörpers vorgespannt.

Wegen der sehr unterschiedlichen und individuellen Größen und Formen der Gliedmaßen von Patienten werden in einer Klinik regelmäßig mehrere Blutsperremanschetten in unterschiedlichen Größen und Formen bereit gestellt. Dies ist aufwendig und teuer. Zur Lösung dieses Problems wird in der US 2013/ 028 96 12 A1 eine preiswerte Blutsperremanschette für einen Einmalgebrauch vorgeschlagen, welche für verschiedene nicht-zylindrische Körperglieder verwendet werden kann. Diese Blutsperremanschette umfasst eine im Wesentlichen bogenförmige äußere Hülle, welche eine aufblasbare Blase enthält. Auf der Außenseite der Hülle ist ein Befestigungsgurt angeordnet, welcher aus einem nicht dehnbaren Material gefertigt ist. Mit diesem Befestigungsgurt wird die in ihren Endabschnitten überlappend um das Körperglied geschlungene Manschette am Körperglied befestigt. Die Hülle ist dabei aus einem textilen Material gebildet, insbesondere einem Nylon-Gestrick oder -Gewebe, welches vor dem Anlegen der Manschette an ein Körperglied sterilisiert wird. Aufgrund der flexiblen Textilstruktur der Manschettenhülle besteht dabei die Gefahr, dass in der Hülle Falten entstehen. Insbesondere beim Aufblasen der Manschette entstehen Falten, da sich der innere Umfang im Vergleich zum äußeren Umfang verkürzt, wodurch ein Materialüberschuss entsteht, der zur Ausbildung von Falten führt. Weiterhin lässt sich die bekannte Blutsperremanschette aus der US 2013/ 028 96 12 A1 nur unzureichend reinigen und sterilisieren und sie ist nur für einen einmaligen Gebrauch vorgesehen, was im Hinblick auf den Anfall von Abfall und unter dem Gesichtspunkt der Ressourceneffizienz nachteilig ist.

Es hat sich ferner gezeigt, dass bei den bekannten Blutsperremanschetten, welche eine Hülle aus einem textilen Material oder aus Gummi aufweisen und zumindest teilweise überlappend um das Körperglied geschlungen werden, eine ungleichmäßige Druckverteilung von der Manschette auf das darunter liegende Körperglied ausgeübt wird. Diese ungleichmäßige Druckverteilung resultiert einerseits aus der ungleichmäßigen Form des Körperglieds, welches in der Regel keine gleichförmige zylindrische Form aufweist, und andererseits aus einer Faltenbildung im textilen Material bzw. dem Gummimaterial beim Anlegen und Aufblasen der Manschette. Um einen möglichst gleichmäßigen Druck auf das Körperglied auszuüben, muss die Manschette absolut glatt um das Körperglied gelegt werden. Falten und Überlappungen erzeugen Druckstellen, die sich später als Spannungsblasen oder Hautnekrosen äußern können.

Eine chirurgische Manschette zum Anlegen an ein Körperglied ist aus der US 2 291 785 B bekannt. Diese Manschette umfasst einen kegelstumpf- und schlauchförmigen Grundkörper, der aus einer inneren und einer äußeren Wand aus einem dünnen, flexiblen und dehnbaren Material zusammen gesetzt ist, wobei die beiden Wände an ihren äußeren Enden miteinander verbunden und im übrigen Bereich im Abstand zueinander verlaufen. An einem Ende sind die beide Wände miteinander verbunden, um einen äußeren Ringabschnitt zu bilden, der sowohl in longitudinaler als auch in transversaler Richtungen flexibel und dehnbar ist. Zwischen den beiden Wänden sind mehre Schichten eines Gewebes angeordnet, welche über einen Gummikleber untereinander und mit den beiden Wänden verbunden sind. An dem anderen Endbereich, der dem äußeren Ringabschnitt gegenüber liegt, sind die Wände mit Textilstreifen verstärkt, so dass der Grundkörper zwar im gesamten Bereich flexibel und biegbar, jedoch nur im Bereich des ringförmigen Abschnitts dehnbar ist. Der inneren Wand liegt radial innen eine Auskleidungswand aus einem flexiblen und dehnbaren Gummimaterial gegenüber, welche an ihren Randbereichen mit den beiden Wänden verbunden ist, so dass sich zwischen der inneren Wand des Grundkörpers und der Auskleidungswand eine abgedichtete, ringförmige Kammer ausbildet, die mittels Druckluft aufgeblasen werden kann. Im drucklosen, nicht aufgeblasenen Zustand verlaufen die Auskleidungswand und die gegenüberliegende innere Wand koaxial zueinander. Wenn Druckluft in die Kammer eingeblasen wird, dehnt sich wegen der Steifigkeit des Grundkörpers im Wesentlichen nur die Auskleidungswand radial nach innen aus. An vier diametral gegenüberliegenden Stellen ist die Auskleidungswand in longitudinaler Richtung an der inneren Wand befestigt, so dass sich an diesen Stellen Einbuchtungen bilden, die einen größeren Widerstand gegen eine Dehnung der Auskleidungswand aufweisen, weshalb sich beim Aufblasen zwischen diesen Einbuchtungen radial nach innen weisende Ausbuchtungen ausbilden, die sich in longitudinaler Richtung erstrecken. Der Grundkörper wird im nicht aufgeblasenen (drucklosen) Zustand an ein Körperglied angelegt und danach aufgeblasen, wobei die radial nach innen anschwellenden Ausbuchtungen auf das Körperglied einen Druck ausüben. Der Vorteil dieser Anordnung liegt darin, dass nicht im gesamten Umfangsbereich des Grundkörpers sondern nur im Bereich der Ausbuchtungen ein Druck ausgeübt wird, der zwar den arteriellen Blutfluss in das Körperglied zulässt, jedoch den venösen Blutfluss weitgehend unterbricht. Bei Blutsperremanchetten ist jedoch ein gleichmäßger Druck auf das Körperglied über den gesamten Umfang erforderlich, um den Blutfluss zu unterbinden und eine Blutsperre in dem Körperglied zu erzeugen.

Hiervon ausgehend liegt der Erfindung die Aufgabe zugrunde, eine Blutsperremanschette zum Anlegen an ein Körperglied aufzuzeigen, welche zuverlässig und einfach zu Reinigen und zu Sterilisieren ist, einfach und schnell an ein Körperglied angelegt werden kann und zuverlässig und dauerhaft eine Blutsperre in dem Körperglied erzeugen kann, wobei eine möglichst homogene Verteilung des von der Blutsperremanschette auf das Körperglied ausgeübten Drucks erzielt und Belastungen des Gewebes und der Haut des Körperglieds möglichst gering gehalten werden sollen.

Diese Aufgaben werden mit einer Blutsperremanschette mit den Merkmalen des Anspruchs 1 sowie dem Verfahren zur Herstellung einer Blutsperremanschette mit den Merkmalen des Anspruchs 15 gelöst. Bevorzugte Ausführungsformen der Blutsperremanschette sind den abhängigen Ansprüchen zu entnehmen.

Die erfindungsgemäße Blutsperremanschette dient zum Anlegen an ein Körperglied und umfasst einen mit einem Druckgas aufblasbaren Aufblaskörper, der eine Aufblaskammer umgibt und aus einem länglichen, sich entlang einer Längsrichtung erstreckenden Träger aus einem biegbaren Trägermaterial und einer auf dem Träger befestigten Dehnungswand aus einem Dehnungsmaterial gebildet ist. Die Dehnungswand ist dabei unter Dehnung des Dehnungsmaterials zumindest in Längsrichtung auf dem länglichen Träger befestigt, der aus einem biegbaren, aber unelastischen Trägermaterial gefertigt ist. Aufgrund der Dehnung der Dehnungswand und der gegenüber dem Dehnungsmaterial geringeren Dehnbarkeit des Trägermaterials übt die Dehnungswand auf den länglichen Träger zumindest in Längsrichtung eine Zugspannung aus, die einerseits dazu führt, dass sich der Träger ringförmig verbiegt und andererseits die Dehnungswand bereits im drucklosen Zustand des Aufblaskörpers in eine konvexe Form auswölbt. Durch die ringförmige Verbiegung des Trägers und die konvexe Auswölbung der Dehnungswand ergibt sich insgesamt bereits im drucklosen Zustand des Aufblaskörpers die Form eines (an den querseitigen Kanten des Trägers unterbrochenen und radial außenseitig ringförmig ausgebildeten) Toroids, wobei der Aufblaskörper bereits im drucklosen Grundzustand (also bei Umgebungsdruck im Aufblaskörper) formstabil und faltenfrei ist.

Sowohl das Trägermaterial als auch das Dehnungsmaterial ist dabei gas- und insbesondere luftdicht. Der Aufblaskörper weist zweckmäßig zwei gegenüberliegende Enden mit querseitigen Kanten und dazwischen in Längsrichtung verlaufende Längskanten auf, wobei die Dehnungswand an ihren Rändern umlaufend und luftdicht mit dem Träger verbunden, insbesondere verklebt oder verschweißt ist, so dass zwischen dem Träger und der Dehnungswand eine gasdichte (luftdichte) Aufblaskammer ausgebildet ist. Diese weist zweckmäßig eine Öffnung auf, an die ein Gasschlauch (Druckluftschlauch) zur Zuführung eines komprimierten Gases (Druckluft) anschließbar ist. Die Öffnung ist zweckmäßig im Bereich einer Längskante oder einer Querkante des Aufblaskörpers angeordnet, damit ein mit der Öffnung verbundener Schlauch nicht beim Verschließen der Manschette (bspw. mittels Klettverschlüsse, welche am Außenumfang der Manschette befestigt werden) stört.

Die Blutsperremanschette wird an ein Körperglied zur Erzeugung eine Blutsperre angelegt, indem die Manschette um das Körperglied geschlungen und die (querseitigen) Enden des Aufblaskörpers im drucklosen Zustand überlappend aufeinander gelegt und mittels Befestigungsmittel, bspw. Haft- oder Klettverschlüsse, aneinander fixiert werden. Bei an einem Körperglied angelegter Blutsperremanschette ist der Aufblaskörper damit torusförmig um das Körperglied gebogen, wobei die Dehnungswand dem Körperglied zugewandt ist und dort zunächst unter leichtem Druck, der durch die Befestigungsmittel fixiert wird, anliegt. Die im nicht aufgeblasenen Grundzustand konvex (vom Träger weg weisend) gewölbte Dehnungswand kann sich dabei durch den von den Befestigungsmitteln fixierten Druck zusammen drücken, bis sie teilweise oder vollflächig auf der Innenfläche des Trägers aufliegt. Dabei wird die im Aufblaskörper im drucklosen Zustand (also bei Umgebungsdruck) noch enthaltene Luft durch die Öffnung und den ggf. damit verbundenen Schlauch heraus gepresst. Aufgrund der Elastizität des Dehnungsmaterials bilden sich dabei keine Falten in dem Dehnungsmaterial aus.

Beim Aufblasen des Aufblaskörpers mittels eines Druckgases (insbesondere Druckluft) wölbt sich die Dehnungswand radial nach innen in Richtung des Körperglieds und übt dadurch einen Druck auf das Körperglied aus. Der von der Blutsperremanschette auf das Körperglied ausgeübte Druck kann - mittels Steuerung des von der Druckgasquelle erzeugten Drucks - so eingestellt werden, dass er deutlich höher liegt als der arterielle systolische Blutdruck des Patienten, um in dem Körperglied eine Blutsperre zu erzeugen. Eine effektive Blutsperre wird bei Patienten mit normalem Blutdruck (RR ≤ 150 mmHg) erreicht, wenn der auf das Körperglied ausgeübte Druck am Arm bei etwa 250 mmHg und am Bein bei etwa 350 mm Hg oder darüber liegt.

Der Träger liegt bei angelegter Manschette radial außen und verhindert aufgrund seiner gegenüber der Dehnungswand geringeren Dehnbarkeit bzw. Elastizität, dass sich der Aufblaskörper beim Aufblasen nennenswert radial nach außen auswölbt. Dadurch kann der für die Erzeugung einer Blutsperre notwendige Druck des Druckgases geringer gehalten werden, wodurch Energie für die Aufrechterhaltung eines hohen Drucks des Druckgases gespart werden kann.

Um ein nennenswertes Auswölben des Aufblaskörpers radial nach außen zu verhindern, ist es vorteilhaft, wenn das Verhältnis des Elastizitätsmoduls des Trägermaterials und des Elastizitätsmoduls des Dehnungsmaterials größer als 10 und bevorzugt größer als 100 ist.

Bevorzugt handelt es sich bei dem Dehnungsmaterial um elastomere Polymere, insbesondere thermoplastische Elastomere (TPE) oder terpolymere Elastomere, insbesondere Kautschuk, Ethylen-Propylen-Dien-Kautschuk (EPDM) oder Ethylen-Propylen-Kautschuk (EPM), Gummimaterialien oder Silikone bzw. Silikonkautschuk. Es können jedoch auch dehnbare Gewebe oder Gestricke, die mit einem luftdichten und dehnbaren Material beschichtet sind, als Dehnungsmaterialien eingesetzt werden. Von Vorteil ist es, wenn das Dehnungsmaterial eine Shore-Härte im Bereich von 17 bis 100 Shore A und/oder einen Elastizitätsmodul von 0,05 bis 10 MPa aufweist.

Der Träger ist bevorzugt aus einer ebenen Kunststoffplatte, insbesondere aus einem thermoplastischen Kunststoff wie Polycarbonat (PC), Polyethylenterephthalat (PET), Polyethylen (PE), Polypropylen (PP), Polystyrol (PS), Polyetheretherketon (PEEK), Polyvinylidenfluorid (PVDF) und Polyvinylchlorid (PVC), oder aus einem Metallblech gefertigt. Von Vorteil ist es, wenn der Träger aus einem sterilisierbaren Trägermaterial gefertigt ist, um eine Sterilisierung und damit eine mehrfache Verwendung der Blutsperremanschette zu ermöglichen.

Die erfindungsgemäße Blutsperremanschette weist damit einen Aufblaskörper auf, der einen ersten Abschnitt in Form der Dehnungswand aus einem elastisch dehnbaren Material (Dehnungsmaterial) und einen radial äußeren zweiten Abschnitt in Form des Trägers aufweist, der starrer ausgebildet ist als der dehnbare erste Abschnitt, so dass sich der Aufblaskörper beim Aufblasen zumindest im Wesentlichen nur radial nach innen in Richtung des Körperglieds, an dem die Blutsperremanschette angelegt ist, und nicht oder allenfalls nur gering radial nach außen aufblähen kann. Der radial äußere zweite Abschnitt ist hierfür im Vergleich zu dem dehnbaren und radial inneren ersten Abschnitt aus einem steiferen (d.h. weniger dehnbarem) Material gebildet, welches sich beim Aufblasen des Aufblaskörpers zumindest weniger verformt (ausdehnt) als der erste Abschnitt (Dehnungswand). Beim Aufblasen des Aufblaskörpers dehnt sich dieser deshalb nur radial nach innen aus und übt dadurch einen Druck auf das in Bezug auf den Aufblaskörper radial innen liegende Körperglied aus. Dabei wird die den radial inneren ersten Abschnitt des Aufblaskörpers bildende Dehnungswand konvex in Richtung des Körperglieds ausgewölbt. Die konvexe Wölbung stellt beim Aufblasen des Aufblaskörpers eine homogene Druckverteilung auf das Körperglied sicher. Durch die konvexe Form passt sich der Aufblaskörper beim Aufblasen optimal an die Form des Körperglieds an. Die Blutsperreeinrichtung kann wegen der Elastizität des Dehnungsmaterials der Dehnungswand in seinem zum Körperglied hin weisenden ersten Abschnitt auch für Körperglieder mit unterschiedlichem Umfang und/oder nichtzylindrischer Form (also bspw. mit im Wesentlichen konischer Form) verwendet oder an unterschiedlichen Stellen eines Körperglieds mit verschiedenem Umfang sowie an unterschiedlichen Körpergliedern mit einer nichtzylindrischen Form angelegt werden. Die Bereitstellung einer Vielzahl von Blutsperremanschetten mit unterschiedlichen Größen und Formen für verschiedene Körperglieder oder Patienten ist daher nicht erforderlich, weil die erfindungsgemäße Blutsperremanschette zumindest für die Standard-Fälle verwendet werden kann.

Der Aufblaskörper der erfindungsgemäßen Blutsperremanschette ist durch die Vorspannung der Dehnungswand auf dem Träger auch im drucklosen (d.h. im nicht aufgeblasenen) Zustand formstabil und selbsttragend. Durch die einerseits flexible und dehnbare Ausgestaltung und andererseits formstabile Grundform des Aufblaskörpers ist dieser im nicht aufgeblasenen Zustand frei von Falten und kann sich beim Aufblasen ausdehnen, ohne dass Falten entstehen. Wegen der Faltenfreiheit des Aufblaskörpers im nicht aufgeblasenen Zustand kann die erfindungsgemäße Blutsperremanschette einfacher für mehrfache Verwendungen aufbereitet werden, bspw. durch eine Reinigung mit einem Desinfektionsmittel oder durch Sterilisierung.

Die schon im nicht aufgeblasenen Zustand formstabile Grundform des Aufblaskörpers gewährleistet darüber hinaus beim Aufblasen eine gleichmäßig über den Innenumfang des Aufblaskörpers verlaufende Verteilung des auf das Körperglied ausgeübten Kompressionsdrucks. Durch die Elastizität des dehnbaren ersten Abschnitts des Aufblaskörpers schmiegt sich dieser beim Aufblasen an die Form des Körperglieds an.

Dadurch werden Druckeinschnürungen im Körperglied an den Stellen mit einem größeren Umfang bzw. Durchmesser vermieden und es wird auch ein Verrutschen der Blutsperremanschette entlang des Körperglieds verhindert. Weiterhin werden dadurch Hautverletzungen am Körperglied vermieden und es ergibt sich während der Dauer der Blutsperre eine geringere Belastung des Gewebes im Körperglied.

Zur Herstellung einer erfindungsgemäßen Blutsperremanschette wird ein sich entlang einer Längsrichtung (L) erstreckender länglicher Träger mit einer vorgegebenen Trägerlänge bereit gestellt, wobei das Trägermaterial gasdicht, insbesondere luftdicht, und biegbar, jedoch nicht oder kaum dehnbar ist. Zweckmäßig hat der Träger die Form eines flachen, rechteckigen Streifens mit entlang der Längsrichtung verlaufenden Längskanten und einer Innenfläche sowie einer Außenfläche. Weiterhin wird eine sich ebenfalls entlang einer Längsrichtung erstreckende längliche, insbesondere streifenförmige, Dehnungswand aus einem gasdichten, insbesondere luftdichten Dehnungsmaterial mit einer vorgegebenen Länge bereit gestellt bzw. auf die Form und Dimension des Trägers zugeschnitten, wobei die Länge der Dehnungswand kürzer als die Trägerlänge ist und die Breite der Dehnungswand zweckmäßig der Breite des Trägers entspricht. Die Dehnungswand weist zweckmäßig ebenfalls eine rechteckige, insbesondere streifenförmige Form mit umlaufenden Randabschnitten auf, welche im Bereich der Längskanten und der Querkanten angeordnet sind. Die Dehnungswand wird unter Dehnung in Längsrichtung auf die Innenfläche des Trägers gelegt, so dass die Dehnungswand mit dem Träger fluchtet. Die Dehnungswand wird dabei soweit gedehnt, dass ihre gedehnte Länge mit der Trägerlänge übereinstimmt. Anschließend werden die Randabschnitte der Dehnungswand gas-, insbesondere luftdicht am Träger befestigt, bspw. Durch Verkleben oder Verschweißen, so dass sich zwischen der Innenfläche des Trägers und der Dehnungswand eine gasdichte Aufblaskammer ausbildet. Durch die Dehnung übt die Dehnungswand auf den Träger eine Zugspannung in Längsrichtung aus, wodurch einerseits das Trägermaterial ringförmig gebogen wird und sich andererseits die Dehnungswand konvex (vom Träger weg) nach außen auswölbt. Dadurch entsteht ein Aufblaskörper, der im Wesentlichen die Form eines (an den Querkanten unterbrochenen) Torus aufweist und eine Aufblaskammer umschließt, wobei die Außenfläche des Trägers radial außen und die konvex gewölbte Dehnungswand radial innen liegt.

Schließlich kann in dem Aufblaskörper, zweckmäßig im Bereich einer Längskante des Trägers, eine Öffnung eingebracht und ein Anschlussstutzen an der Öffnung angeordnet werden, über den der Aufblaskörper mit einem Druckschlauch verbindbar ist, um ein Druckgas in die Aufblaskammer einleiten zu können. Statt eines Anschlussstutzens kann auch direkt ein mit einer Druckgasquelle verbindbarer Schlauchabschnitt an der Öffnung angeordnet werden. Auf der Außenfläche des Trägers wird schließlich zweckmäßig ein Befestigungsmittel, bspw. ein Klettverschluss- oder ein Adhäsionsband befestigt, mit dem der Aufblaskörper an einem Körperglied fixiert werden kann.

Die Aufblaskammer kann in mehrere Kammern unterteilt sein, welche durch Kammerwände voneinander getrennt sind. Die Kammerwände sind dabei an der Innenfläche des Trägers angeformt und erstrecken sich - bei ringförmig gebogener Manschette - zwischen der Innenfläche des Trägers und der Dehnungswand in radialer Richtung und können bspw. aus einem dehn- und komprimierbaren Kunststoff- oder Gummimaterial gebildet sein. Zweckmäßig sind die Kammerwände an ihrer radial nach innen weisenden Außenkante, welche an der Dehnungswand anliegt und zweckmäßig mit dieser verbunden ist, kreisförmig abgerundet und dadurch an die konvexe Form der Dehnungswand angepasst. Benachbarte Kammern sind dabei bevorzugt über Durchlässe in der die beiden Kammern trennenden Kammerwand miteinander strömungswirksam verbunden. Ein über eine äußere Öffnung in eine der Kammern eintretendes Druckgas (Druckluft) kann sich durch diese Durchlässe in den Kammerwänden gleichmäßig in den einzelnen Kammern verteilen, so dass in allen Kammern ein gleichförmiger Druck entsteht.

Der dehnbare und von der Dehnungswand gebildete erste Abschnitt des Aufblaskörpers erfüllt in dieser Ausführungsform den Zweck, dass sich die Kammern innerhalb der vorgegebenen Form des dehnbaren Abschnitts ausdehnen können. In dieser Ausführungsform wird die konvexe Grundform der Dehnungswand (die diese bereits aufgrund der Vorspannung auf dem Träger einnimmt) durch die Kammerwände unterstützt und insbesondere im drucklosen Zustand aufrecht erhalten.

Diese und weitere Vorteile und Eigenschaften der erfindungsgemäßen Blutsperreeinrichtung gehen aus dem nachfolgend unter Bezugnahme auf die begleitenden Zeichnungen näher beschriebenen Ausführungsbeispiel hervor. Die Zeichnungen zeigen:
- **Figur 1a**:: Perspektivische Darstellung einer erfindungsgemäßen Blutsperremanschette;
- **Figur 1b:**: Darstellung der erfindungsgemäßen Blutsperremanschette von Figur 1a in einer Seitenansicht,
- **Figur 2:**: Darstellung der Blutsperremanschette von Figur 1 in einer Draufsicht (Figur 2a) und einer Detailansicht im Bereich der Leitung, mit der die Blutsperremanschette an eine Druckgasquelle anschließbar ist (Figur 2b);
- **Figur 3:**: schematische Darstellung des Aufbaus und der Herstellung einer erfindungsgemäßen Blutsperremanschette, wobei Figur 3a den Träger, Figur 3b die (ungedehnte) Dehnungswand und Figur 3c eine Seitenansicht des aus dem Träger und der darauf unter Dehnung befestigten Dehnungswand zeigt.

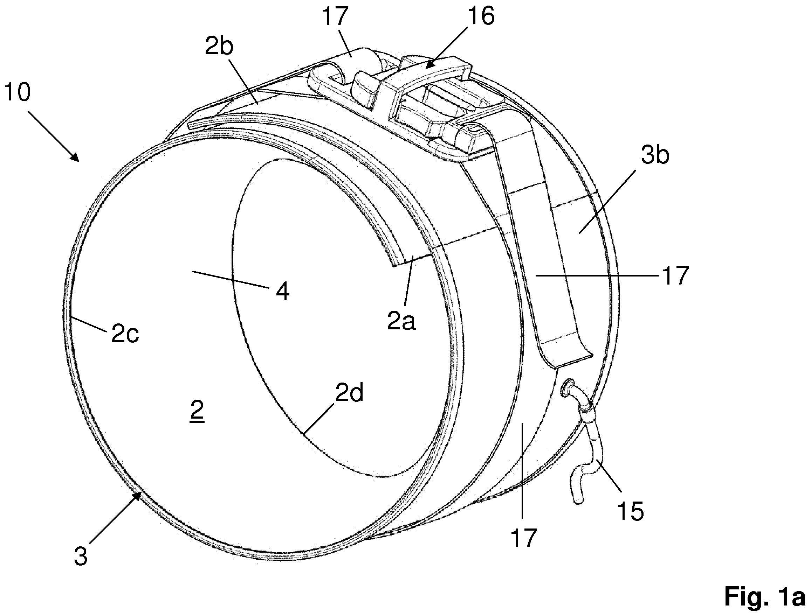

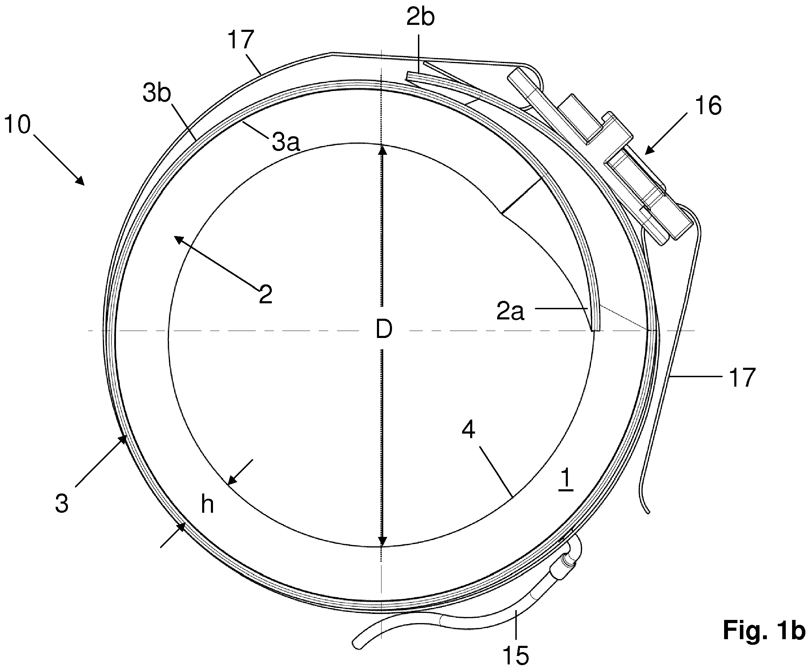

In den Figuren 1a und 1b ist eine erfindungsgemäße Blutsperremanschette 10 zum Anlegen an ein Körperglied dargestellt. Die Blutsperremanschette 10 umfasst einen Aufblaskörper 2, der eine luftdichte Aufblaskammer 1 umgibt. Der Aufblaskörper 2 ist über eine Leitung 15, welche mit der Aufblaskammer 1 in Verbindung steht, mit einer hier nicht gezeigten Druckgasquelle verbindbar. Bei der Druckgasquelle kann es sich bspw. um einen Kompressor handeln, der ein Druckgas, insbesondere Druckluft bereit stellt, mit dem der Aufblaskörper aufgeblasen werden kann. Der Druck, mit dem der Aufblaskörper 2 aufgeblasen wird, wird dabei über die in der Druckgasquelle enthaltene Steuereinheit gesteuert. Derartige Steuereinheiten zur Steuerung von Blutsperremanschetten sind aus dem Stand der Technik bekannt. Auf der Außenseite des Aufblaskörpers 2 ist ein Verschlussband 17 mit einem Verschluss 16 befestigt. Das Verschlussband 17 kann mit dem Verschluss 16, der hier als Klickverschluss ausgebildet ist, in einer gewünschten Position fixiert werden, um die Blutsperremanschette 10 eng an einem Körperglied anliegend befestigen zu können. Statt des Verschlusses 16 kann das Verschlussband auch als Klettband ausgebildet sein.

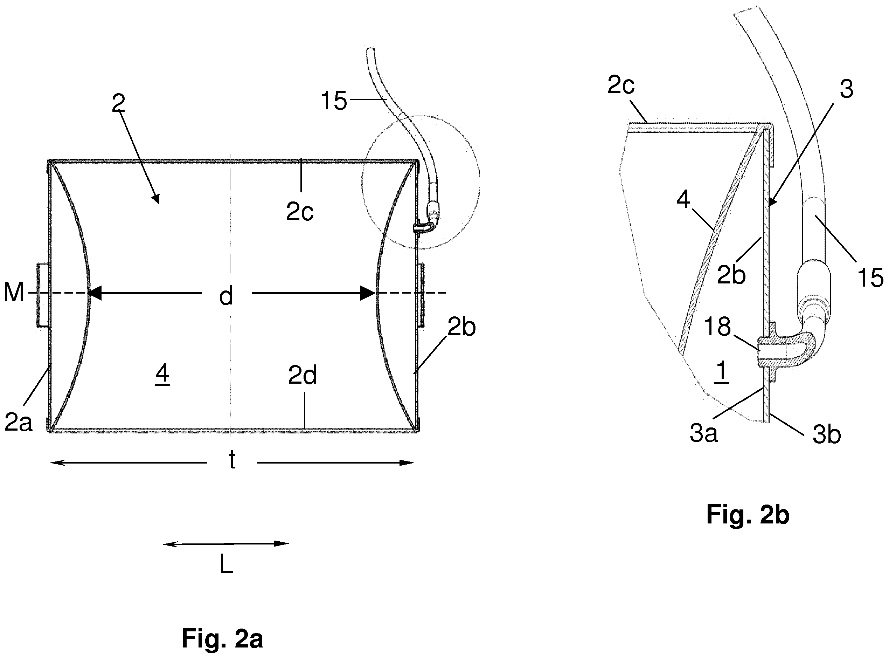

In Figur 2 ist die Blutsperremanschette 10 von Figur 1 in einer Draufsicht (Figur 2a) und einer Detailansicht im Schnitt im Bereich der Leitung 15 (Figur 2b) gezeigt.

Wie aus Figur 2a ersichtlich, setzt sich die Aufblaskammer 2 aus einem länglichen, sich entlang einer Längsrichtung L erstreckenden Träger 3 aus einem biegbaren Trägermaterial und einer auf dem Träger 3 befestigten Dehnungswand 4 aus einem Dehnungsmaterial zusammen. Die Dehnungswand 4 ist unter Dehnung des Dehnungsmaterials zumindest in Längsrichtung auf dem länglichen Träger 3 befestigt.

Zur Ausbildung einer gas- bzw. luftdichten Aufblaskammer 1 zwischen dem Träger 3 und der Dehnungswand 4 ist sowohl das Trägermaterial als auch das Dehnungsmaterial gas- bzw. luftdicht ausgebildet und die Dehnungswand 4 ist in einer gas- bzw. luftdichten Verbindung am Träger 3 befestigt, insbesondere Verklebt oder Verschweißt.

Bei dem Trägermaterial des Trägers 3 handelt es sich um ein biegbares aber zumindest im Wesentlichen unelastisches Material, beispielsweise um einen thermoplastischen Kunststoff wie PE, PP oder bevorzugt um Polyvinylidenfluorid (PVDF), oder um ein Metallblech. Das Trägermaterial ist zumindest wesentlich weniger dehnbar als das Dehnungsmaterial aus dem die Dehnungswand 4 gefertigt ist.

Bei dem Dehnungsmaterial der Dehnungswand 4 handelt es sich um ein elastisches (dehnbares) Material, beispielsweise um ein Gummi-Material oder eine dehnbare Kunststofffolie. Bei dem Dehnungsmaterial kann es sich auch um ein Textilmaterial (beispielsweise Polyester) handeln, welches mit einer luftdichten Beschichtung (beispielsweise aus Polyurethan) beschichtet ist. Die Beschichtung kann dabei ein- oder zweiseitig auf dem Textilmaterial aufgebracht sein. Das Dehnungsmaterial kann uniaxial oder biaxial dehnbar sein. Im Falle der Verwendung eines biaxial dehnbaren Dehnungsmaterials kann die Dehnungswand 4 zusätzlich zur Längsrichtung L auch in Querrichtung gedehnt auf dem Träger 3 befestigt sein, wobei es allerdings bevorzugt ist, die Dehnungswand 4 nur in Längsrichtung L zu dehnen.

Die Dehnung des Dehnungsmaterials liegt bevorzugt bei mindestens 250 % und kann zweckmäßig eine Dehnung des Dehnungsmaterials bis zu 450 % ermöglichen. Zweckmäßig weißt das Dehnungsmaterial eine Shore-Härte im Bereich von 17 bis 100 Shore A sowie einen Elastizitätsmodul im Bereich von 0,05 bis 10 MPa auf. Der Elastizitätsmodul des Dehnungsmaterials ist dabei wesentlich kleiner als der Elastizitätsmodul des Trägermaterials. Zweckmäßig beträgt das Verhältnis des Elastizitätsmoduls des Trägermaterials und des Elastizitätsmoduls des Dehnungsmaterials mehr als 10 und bevorzugt mehr als 100.

Aufgrund der Dehnung der Dehnungswand 4 und der gegenüber dem Dehnungsmaterial wesentlich geringeren Dehnbarkeit des Trägermaterials übt die Dehnungswand 4 auf den länglichen Träger 3 zumindest in Längsrichtung L (und ggf. auch in Querrichtung, wenn die Dehnungswand auch quer zur Längsrichtung auf dem Träger 3 vorgespannt ist) eine Zugspannung aus. Diese führt einerseits dazu, dass sich der Träger 3 - wie in den Figuren 1 und 2b gezeigt - ringförmig verbiegt. Andererseits führt die Dehnung der Dehnungswand 4 auf dem Träger 3 zu einer konvexen Auswölbung der Dehnungswand 4 (konvexe Wölbung der Dehnungswand 4 in Bezug auf die Oberfläche des Trägers 3), wie insbesondere aus Figur 2b ersichtlich. Durch die ringförmige Verbiegung des Trägers 3 und die konvexe Auswölbung der Dehnungswand 4 ergibt sich bereits im drucklosen Zustand des Aufblaskörpers (also bei Umgebungsdruck in der Aufblaskammer 1) die aus den Figuren ersichtliche Form eines an den querseitigen Kanten des Trägers unterbrochenen und radial außenseitig ringförmig ausgebildeten Toroids. Der Aufblaskörper 2 ist dadurch bereits im drucklosen Grundzustand formstabil und weist insbesondere im Bereich der Dehnungswand 4 aufgrund deren Dehnung keine Falten auf.

In dem zeichnerisch dargestellten Ausführungsbeispiel einer erfindungsgemäßen Blutsperremanschette weist der Aufblaskörper 2 zwei gegenüberliegende Enden 2a, 2b mit querseitigen Kanten und dazwischen in Längsrichtung L verlaufende Längskanten 2c, 2d auf (Figur 2a). Die Dehnungswand 4 ist dabei an ihren Rändern umlaufend und luftdicht mit dem Träger 3 verbunden, beispielsweise durch Verklebung oder Verschweißung. Die Dehnungswand 4 liegt dabei einer Innenfläche 3a des Trägers 3 gegenüber (Figur 2b). Zwischen der Innenfläche 3a des Trägers 3 und der (gedehnten) Dehnungswand 4 erstreckt sich die Aufblaskammer 1, welche aufgrund der luftdichten Ausbildung des Trägermaterials und des Dehnungsmaterials sowie der luftdichten Befestigung der Dehnungswand 4 am Träger 3 gas- bzw. luftdicht ausgebildet ist. In der Aufblaskammer 2 ist zweckmäßig im Bereich einer Längskante des Trägers 3 eine Öffnung 18 vorgesehen, an die eine Leitung 15, bspw. ein Druckluftschlauch, angeschlossen ist, welche mit einer Druckgasquelle verbunden werden kann, um die Aufblaskammer 2 mit Druckgas, insbesondere Druckluft, zu beaufschlagen (Figur 2b).

An der Außenfläche 3b des Trägers 3 ist ein Befestigungsmittel angeordnet. Bei dem Befestigungsmittel kann es sich beispielsweise um ein Klettverschluss- oder ein Adhäsionsband handeln, welches auf der Außenfläche 3b des Trägers durch Klett- oder Adhäsionswirkung befestigbar ist. In dem zeichnerisch dargestellten Ausführungsbeispiel umfassen die Befestigungsmittel ein nicht oder nur wenig dehnbares Verschlussband 17 mit einem Klickverschluss 16, mit dem das Verschlussband in einer gewünschten Position fixierbar ist (Figur 1).

Der Draufsicht von Figur 2a ist zu entnehmen, dass die gegenüberliegenden Enden 2a, 2b des Aufblaskörpers 2 überlappen, wodurch ein insgesamt und im Wesentlichen torusförmiger Aufblaskörper 2 gebildet wird. Der Innenumfang des torusförmig gebogenen Aufblaskörpers 2 umgibt dabei einen Durchgang mit einem lichten Innendurchmesser D, der im Bereich der äußeren Längskanten 2c, 2d des Aufblaskörpers 2 am größten und im Bereich der Mittellängsachse M am kleinsten ist. Dieser Verlauf des lichten Innendurchmessers des Aufblaskörpers 2 ergibt sich durch die bereits im drucklosen Zustand der Blutsperremanschette 2 ausgebildete konvexe Auswölbung der Dehnungswand 4 in radialer Richtung nach innen. Im Bereich der Mittellängsachse M des Aufblaskörpers 2 weist die konvex vom Träger 3 weg gewölbte Dehnungswand 4 zur Innenfläche 3a des Trägers 3 einen (maximalen) Abstand h auf, der zweckmäßig im Bereich von 1 bis 3 cm und bspw. bei 2 cm liegt. Der genaue Abstand h hängt dabei von den Dehnungseigenschaften des Dehnungsmaterials ab und kann bei der Herstellung an den vorgesehenen Einsatzzweck der Blutsperremanschette angepasst werden.

Zur Erzeugung einer Blutsperre in einem Körperglied wird die erfindungsgemäße Blutsperremanschette an das Körperglied angelegt, indem die (bereits im drucklosen Zustand torusförmig gebogene) Aufblaskammer 2 um das Körperglied geschlungen wird, wobei sich die gegenüberliegenden Enden 2a, 2b des Aufblaskörpers 2 überlappen. Der Aufblaskörper 2 wird durch die an der Außenfläche 3b des Trägers 3 angeordneten Befestigungsmittel unter Zugspannung am Körperglied fixiert. Die Zugspannung wird dabei zweckmäßig so gewählt, dass die Dehnungswand 4 soweit in Richtung der Innenfläche 3a des Trägers 3 zusammengedrückt wird, dass diese zumindest teilweise, bevorzugt vollständig an der Innenfläche 3a an- bzw. aufliegt. Der auf diese Weise an dem Körperglied fixierte Aufblaskörper 2 wird anschließend mit einem Druckgas, insbesondere Druckluft, beaufschlagt, welches über die Leitung 15 in die Aufblaskammer 1 einströmt. Durch das Einströmen des Druckgases in die Aufblaskammer 1 wölbt sich die Dehnungswand 4 radial nach innen aus und übt dadurch einen Kompressionsdruck auf das innenliegende Körperglied aus. Aufgrund der Elastizität des Dehnungsmaterials und der Starrheit des Trägermaterials ist dabei gewährleistet, dass der Aufblaskörper 2 im Wesentlichen nur radial nach innen (und nicht auch radial nach außen) expandiert. Der Druck des in die Aufblaskammer 1 strömenden Druckgases wird mittels der Steuereinrichtung so gesteuert, dass über einen vorgegebenen Zeitraum eine Blutsperre in dem Körperglied erzeugt wird.

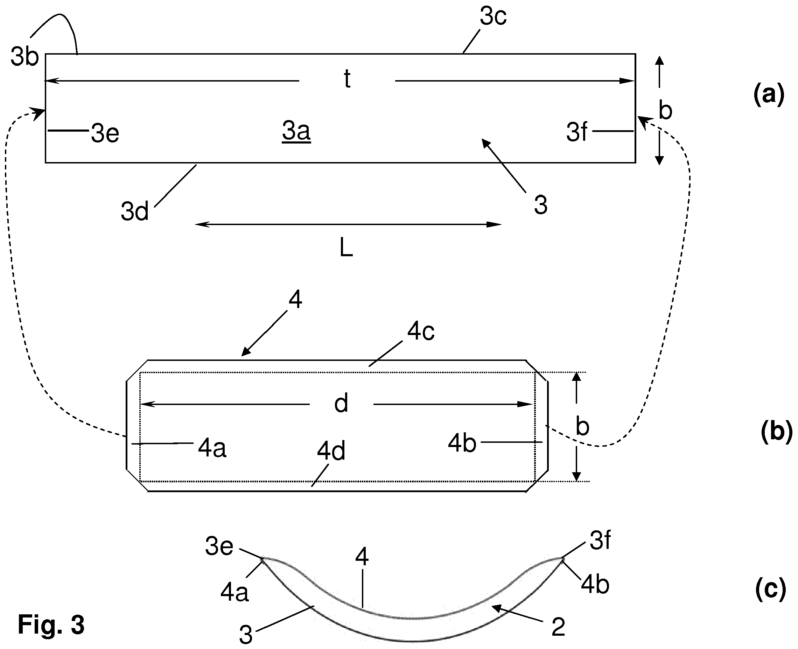

Zur Herstellung einer erfindungsgemäßen Blutsperremanschette 10 wird ein länglicher, sich entlang einer Längsrichtung L erstreckender Träger 3 aus einem biegbaren und luftdichten, jedoch nicht oder zumindest kaum dehnbaren Trägermaterial verwendet. Zweckmäßig hat der Träger 3 die Form eines rechteckigen Streifens mit zwei in Längsrichtung L verlaufenden parallelen Längskanten 3c, 3d, die eine Trägerlänge t definieren und zwei senkrecht dazu angeordneten Querkanten 3e, 3f, die eine Trägerbreite b definieren, wobei der Träger 3 über eine Innenfläche 3a und eine Außenfläche 3b verfügt. Auf den Träger 3 wird eine ebenfalls längliche Dehnungswand 4 aus einem Dehnungsmaterial gelegt. Die Dehnungswand 4 wird hierfür zweckmäßig ebenfalls als länglicher Streifen ausgebildet und so zugeschnitten, dass ihre ungedehnte Länge d kürzer als die Trägerlänge t ist, wie in Figur 3 gezeigt. Die Breite der Dehnungswand 4 kann der Trägerbreite b entsprechen oder - bei Verwendung eines biaxial dehnbaren Dehnungsmaterials - (geringfügig) kleiner sein.

Die Dehnungswand 4 weist zweckmäßig sowohl in ihrer Längsrichtung L als auch in ihrer Querrichtung umlaufende Randabschnitte 4a, 4b, 4c, 4d auf, die sich an die (ungedehnte) Länge d bzw. die Breite b der Dehnungswand anschließen. Die Dehnungswand 4 wird auf die Innenfläche 3a des Trägers 3 gelegt (wie mit den gestrichelten Pfeilen in Figur 3 angedeutet) und unter Dehnung des Dehnungsmaterials in Längsrichtung L auf die Trägerlänge t am Träger 3 befestigt, indem die Randabschnitte 4a - 4d am Träger 3 angeklebt oder angeschweißt werden. Zweckmäßig werden die Randabschnitte 4a - 4d der Dehnungswand 4 so umgefaltet und um den Träger 3 gelegt, dass sie auf dessen Außenfläche 3b verklebt oder verschweißt werden können. Beim Befestigen der Dehnungswand 4 auf dem Träger 3 wird die Dehnungswand 4 (aufgrund der gegenüber der Trägerlänge t geringeren Länge d) gedehnt. Durch das Befestigen der Randabschnitte 4a - 4d und der sich dadurch ergebenden Vorspannung der Dehnungswand 4 auf dem Träger 3 wölbt sich die Dehnungswand 4 konvex von der Innenfläche 3a des Trägers weg. Dies ist in Figur 3c angedeutet. Zwischen der Innenfläche 3a des Trägers 3 und der (konvex gewölbten) Dehnungswand 4 bildet sich dadurch eine luftdichte Aufblaskammer 1 aus, welche sich zwischen den gegenüberliegenden Enden 2a, 2b des durch den Träger 3 und die Dehnungswand 4 gebildeten Aufblaskörpers 2 erstreckt. Durch die Dehnung der Dehnungswand 4 erzeugt diese auf den biegbaren Träger 3 eine Zugspannung, welche dazu führt, dass sich der Träger 3 kreisförmig verbiegt, wobei der Radius der kreisförmigen Verbiegung von der Zugspannung abhängt, die die gedehnte Dehnungswand 4 auf den Träger 3 ausübt. Zweckmäßig ist diese Zugspannung so groß, dass die gegenüberliegenden Enden 2a, 2b des Aufblaskörpers 2 auf Stoß aneinanderliegen oder sich überlappen. Dadurch bildet sich ein im Wesentlichen torusförmig gebogener Aufblaskörper 2 aus, wobei die durch die Biegung ringförmig ausgebildete Außenfläche 3b des Trägers 3 radial außen und die gedehnte Dehnungswand 4 radial innen liegt.

Zum Anschluss der Leitung 15, insbesondere eines Druckluftschlauchs, wird in den Aufblaskörper 2 eine Öffnung 18 eingebracht. Die Öffnung 18 kann bereits bei der Fertigung des Trägers 3 aus dem Trägermaterial in diesem als Einschnitt ausgeformt oder als Bohrung eingebracht werden. Zweckmäßig ist die Öffnung 18 im Bereich einer Längskante oder einer querseitigen Kante an einem Ende des Aufblaskörpers 2 angeordnet. An diese Öffnung 18 wird ein Anschlussstutzen für eine Leitung 15 oder ein an einer Druckgasquelle anschließbares Schlauchstück angebracht. Schließlich wird auf der Außenfläche 3b des Trägers 3 ein Befestigungsmittel, beispielsweise in Form eines Verschlussbands 17 mit einem Verschluss 16 oder ein Klettverschlussband, befestigt.

## Patentansprüche

1. Blutsperremanschette zum Anlegen an ein Körperglied, mit einem eine Aufblaskammer (1) umgebenden Aufblaskörper (2), der aus einem drucklosen Grundzustand mit einem Druckgas aufblasbar ist, um in dem Körperglied eine Blutsperre zu erzeugen, und einem Befestigungsmittel (16, 17) zum Fixieren der Blutsperremanschette am Körperglied, wobei der Aufblaskörper (2) aus einem länglichen, sich entlang einer Längsrichtung (L) erstreckenden Träger (3) aus einem gasdichten und biegbaren Trägermaterial und einer auf dem Träger (3) befestigten Dehnungswand (4) aus einem gasdichten Dehnungsmaterial gebildet ist, **dadurch gekennzeichnet, dass** die Dehnungswand (4) bereits in dem drucklosen Grundzustand des Aufblaskörpers (2) zumindest in Längsrichtung (L) des Trägers (3) gedehnt ist.

2. Blutsperremanschette nach Anspruch 1, **dadurch gekennzeichnet, dass** der Aufblaskörper (2) bei angelegter Blutsperremanschette torusförmig gebogen ist, wobei der Träger (3) radial außen liegt und die Dehnungswand (4) am Körperglied anliegt.

3. Blutsperremanschette nach einem der voranstehenden Ansprüche, **dadurch gekennzeichnet, dass** der Aufblaskörper (2) zwei gegenüberliegende Enden (2a, 2b) und dazwischen in Längsrichtung (L) verlaufende Längskanten (2c, 2d) aufweist, wobei die Dehnungswand (4) an beiden Enden (2a, 2b) und entlang der Längskanten (2c, 2d) luftdicht am Träger (3) befestigt, insbesondere verklebt oder verschweißt ist.

4. Blutsperremanschette nach einem der voranstehenden Ansprüchen, **dadurch gekennzeichnet, dass** das Trägermaterial biegbar ist.

5. Blutsperremanschette nach einem der voranstehenden Ansprüche, **dadurch gekennzeichnet, dass** das Verhältnis des Elastizitätsmoduls des Trägermaterials und des Elastizitätsmoduls des Dehnungsmaterials größer als 10, bevorzugt größer als 100 ist.

6. Blutsperremanschette nach Anspruch 4, **dadurch gekennzeichnet, dass** der lichte Innendurchmesser (D) des Aufblaskörpers (2) im Bereich der beiden Längskanten (2c, 2d) am größten und im Bereich der Mittellängsachse (M) am kleinsten ist.

7. Blutsperremanschette nach einem der voranstehenden Ansprüche, **dadurch gekennzeichnet, dass** die Dehnungswand (4) durch ihre Dehnung unter ringförmiger Verbiegung des Trägers (3) konvex gekrümmt ist und im drucklosen Grundzustand im Bereich der Mittellängsachse (M) vom Träger (3) einen maximalen Abstand (h) aufweist, der im Bereich von 1 bis 3 cm und bevorzugt bei 2 cm liegt.

8. Blutsperremanschette nach einem der voranstehenden Ansprüche, **dadurch gekennzeichnet, dass** sich die Aufblaskammer (1) zwischen einem ersten Abschnitt und einem zweiten Abschnitt des Aufblaskörpers (2) erstreckt, wobei der erste Abschnitt (1a) von der Dehnungswand (4) und der zweite Abschnitt (1b) von dem Träger (3) gebildet ist.

9. Blutsperremanschette nach einem der voranstehenden Ansprüche, **dadurch gekennzeichnet, dass** das Dehnungsmaterial aus der Gruppe ausgewählt ist, welche elastomere Polymere, insbesondere thermoplastische Elastomere (TPE) und terpolymere Elastomere, insbesondere Kautschuk, Ethylen-Propylen-Dien-Kautschuk (EPDM) und Ethylen-Propylen-Kautschuk (EPM), Gummi und Silikone, sowie dehnbare Gewebe oder Gestricke, die mit einem luftdichten und dehnbaren Material beschichtet sind, umfasst.

10. Blutsperremanschette nach einem der voranstehenden Ansprüche, **dadurch gekennzeichnet, dass** das Dehnungsmaterial eine Shore-Härte im Bereich von 17 bis 100 Shore A und/oder einen Elastizitätsmodul von 0,05 bis 10 MPa aufweist.

11. Blutsperremanschette nach einem der voranstehenden Ansprüchen, **dadurch gekennzeichnet, dass** die Dehnungswand (4) sowohl im drucklosen Grundzustand als auch im aufgeblasenen Zustand des Aufblaskörpers im Querschnitt konvex gewölbt ist.

12. Blutsperremanschette nach einem der voranstehenden Ansprüche, **dadurch gekennzeichnet, dass** der Aufblaskörper (2) wenigstens eine Öffnung aufweist, welche mit einer Leitung (15) verbindbar ist, um den Aufblaskörper (2) mit einer Druckgasquelle zu verbinden, wobei die Öffnung vorzugsweise im Bereich einer Längskante (2c, 2d) oder einer querseitigen Kante des Aufblaskörpers (2) angeordnet ist.

13. Blutsperremanschette nach einem der voranstehenden Ansprüche, **dadurch gekennzeichnet, dass** der Träger (3) aus einer ebenen Kunststoffplatte, insbesondere aus einem thermoplastischen Kunststoff, oder aus einem Metallblech gefertigt ist.

14. Blutsperremanschette nach einem der voranstehenden Ansprüche, **dadurch gekennzeichnet, dass** der Aufblaskörper (2) bereits im drucklosen Grundzustand formstabil und faltenfrei sowie aufgrund der Vorspannung der Dehnungswand (4) auf dem Träger (3) torusförmig gebogen ist, wobei der Träger (3) radial außen liegt und aufgrund einer durch die gedehnte Dehnungswand (4) erzeugten Zugspannung ringförmig gebogen ist und die zwei gegenüberliegenden Enden (2a, 2b) des Aufblaskörpers (2) sich überlappen oder stoßförmig aneinander anliegen.

15. Verfahren zur Herstellung einer Blutsperremanschette, welche einen Aufblaskörper (2) enthält, der aus einem länglichen, sich entlang einer Längsrichtung erstreckenden Träger (3) aus einem gasdichten und biegbaren Trägermaterial und einer auf dem Träger (3) befestigten Dehnungswand (4) aus einem gasdichten Dehnungsmaterial gebildet ist, wobei das Verfahren folgende Schritte umfasst:
- Bereitstellen eines sich entlang der Längsrichtung (L) erstreckenden länglichen Trägers (3) mit einer vorgegebenen Trägerlänge (t),
- Bereitstellen einer sich entlang einer Längsrichtung (L) erstreckenden länglichen Dehnungswand (4) mit einer vorgegebenen Länge (d), wobei die Länge (d) der Dehnungswand (4) kürzer als die Trägerlänge (t) ist und die Dehnungswand (4) umlaufende Randabschnitte (4a, 4b, 4c, 4d) aufweist,
- Befestigen der Randabschnitte (4a, 4b, 4c, 4d) der Dehnungswand (4) unter Dehnung des Dehnungsmaterials zumindest in Längsrichtung (L) auf dem länglichen Träger (3), so dass sich zwischen dem Träger und der Dehnungswand eine gasdichte Aufblaskammer (1) ausbildet.

## Claims

1. Tourniquet cuff for application to a limb, having an inflatable body (2) surrounding an inflatable chamber (1) and which is inflatable from a pressure-less base state using a compressed gas in order to generate a blood restriction in the limb, and an attachment means (16, 17) for fixing the tourniquet cuff to the limb, wherein the inflatable body (2) is formed from an elongated support (3) extending along a longitudinal direction (L) and made from a gastight and flexible support material and a stretchable wall (4) attached to the support (3) and made from a gastight stretchable material, **characterised in that** the stretchable wall (4) is stretched at least in the longitudinal direction (L) of the support (3) even in the pressure-less base state of the inflatable body (2).

2. Tourniquet cuff according to claim 1, **characterised in that** the inflatable body (2) is curved like a torus when the tourniquet cuff is applied, wherein the support (3) lies radially outside and the stretchable wall (4) rests on the limb.

3. Tourniquet cuff according to one of the preceding claims, **characterised in that** the inflatable body (2) has two opposite ends (2a, 2b) and longitudinal edges (2c, 2d) running in-between in the longitudinal direction (L), wherein the stretchable wall (4) is attached, in particular adhered or heat-sealed, in airtight manner to the support (3) at both ends (2a, 2b) and along the longitudinal edges (2c, 2d).

4. Tourniquet cuff according to one of the preceding claims, **characterised in that** the support material is flexible.

5. Tourniquet cuff according to one of the preceding claims, **characterised in that** the ratio of the modulus of elasticity of the support material and of the modulus of elasticity of the stretchable material is greater than 10, preferably greater than 100.

6. Tourniquet cuff according to claim 4, **characterised in that** the clear internal diameter (D) of the inflatable body (2) is greatest in the region of the two longitudinal edges (2c, 2d) and smallest in the region of the central longitudinal axis (M).

7. Tourniquet cuff according to one of the preceding claims, **characterised in that** the stretchable wall (4) is curved convexly by stretching thereof with annular deformation of the support (3) and in the pressure-less base state has a maximum distance (h), which lies in the range from 1 to 3 cm and preferably at 2 cm, in the region of the central longitudinal axis (M) of the support (3).

8. Tourniquet cuff according to one of the preceding claims, **characterised in that** the inflatable chamber (1) extends between a first section and a second section of the inflatable body (2), wherein the first section (1a) is formed by the stretchable wall (4) and the second section (1b) by the support (3).

9. Tourniquet cuff according to one of the preceding claims, **characterised in that** the stretchable material is selected from the group comprising elastomeric polymers, in particular thermoplastic elastomers (TPE) and terpolymeric elastomers, in particular rubber, ethylene-polypropylene-diene rubber (EPDM) and ethylene-propylene rubber (EPM), rubber and silicones, and stretchable fabric or knitted fabrics which are coated with an airtight and stretchable material.

10. Tourniquet cuff according to one of the preceding claims, **characterised in that** the stretchable material has a Shore hardness in the range from 17 to 100 Shore A and/or a modulus of elasticity of 0.05 to 10 MPa.

11. Tourniquet cuff according to one of the preceding claims, **characterised in that** the stretchable wall (4) is arched to be convex in cross-section both in the pressure-less base state and in the inflated state of the inflatable body.

12. Tourniquet cuff according to one of the preceding claims, **characterised in that** the inflatable body (2) has at least one opening which is connectable to a pipe (15) in order to connect the inflatable body (2) with a compressed gas source, wherein the opening is preferably arranged in the region of a longitudinal edge (2c, 2d) or a transverse-side edge of the inflatable body (2).

13. Tourniquet cuff according to one of the preceding claims, **characterised in that** the support (3) is manufactured from a flat plastic plate, in particular from a thermoplastic material, or from a metal sheet.

14. Tourniquet cuff according to one of the preceding claims, **characterised in that** the inflatable body (2) is curved like a torus even in the pressure-less base state to be dimensionally stable and fold-free and due to the pretension of the stretchable wall (4) on the support (3), wherein the support (3) lies radially outside and is curved to be annular due to a tensile stress generated by the stretched stretchable wall (4) and the two opposite ends (2a, 2b) of the inflatable body (2) overlap or rest abutting against one another.

15. Method for producing a tourniquet cuff which contains an inflatable body (2) which is formed from an elongated support (3) extending along a longitudinal direction and made of a gastight and flexible support material and a stretchable wall (4) attached to the support (3) and made of a gastight stretchable material, wherein the method comprises the following steps:
- providing an elongated support (3) extending along the longitudinal direction (L) with a predetermined support length (t),
- providing an elongated stretchable wall (4) extending along a longitudinal direction (L) with a predetermined length (d), wherein the length (d) of the stretchable wall (4) is shorter than the support length (t) and has edge sections (4a, 4b, 4c, 4d) surrounding the stretchable wall (4),
- attaching the edge sections (4a, 4b, 4c, 4d) of the stretchable wall (4) with stretching of the stretchable material at least in the longitudinal direction (L) to the elongated support (3) so that a gastight inflatable chamber (1) is formed between the support and the stretchable wall.

## Revendications

1. Manchon de garrottage destiné à être posé au niveau d'un membre du corps, avec un corps gonflable (2) entourant une chambre gonflable (1), qui peut être gonflé avec un gaz sous pression à partir d'un état de base sans pression pour produire dans le membre du corps un garrot, et un moyen de fixation (16, 17) servant à bloquer le manchon de garrottage au niveau du membre de corps, dans lequel
le corps gonflable (2) est formé à partir d'un support (3) allongé s'étendant le long d'un sens longitudinal (L), composé d'un matériau de support étanche aux gaz et pouvant être cintré, et d'une paroi expansible (4) fixée sur le support (3), composée d'un matériau expansible étanche aux gaz, **caractérisé en ce que** la paroi expansible (4) est étirée déjà dans l'état de base sans pression du corps gonflable (2) au moins dans le sens longitudinal (L) du support (3).

2. Manchon de garrottage selon la revendication 1, **caractérisé en ce que** le corps gonflable (2) est cintré en forme toroïdale lorsque le manchon de garrottage est placé, dans lequel le support (3) se situe radialement à l'extérieur et la paroi expansible (4) repose au niveau du membre du corps.

3. Manchon de garrottage selon l'une quelconque des revendications précédentes, **caractérisé en ce que** le corps gonflable (2) présente deux extrémités (2a, 2b) se faisant face et des arêtes longitudinales (2c, 2d) s'étendant de manière intercalée dans le sens longitudinal (L), dans lequel la paroi expansible (4) est fixée, en particulier collée ou soudée, au niveau des deux extrémités (2a, 2b) et le long des arêtes longitudinales (2c, 2d) de manière étanche à l'air au niveau du support (3).

4. Manchon de garrottage selon l'une quelconque des revendications précédentes, **caractérisé en ce que** le matériau de support peut être cintré.

5. Manchon de garrottage selon l'une quelconque des revendications précédentes, **caractérisé en ce que** le rapport entre le module d'élasticité du matériau de support et le module d'élasticité du matériau expansible est supérieur à 10, de manière préférée est supérieur à 100.

6. Manchon de garrottage selon la revendication 4, **caractérisé en ce que** le petit diamètre intérieur (D) du corps gonflable (2) est le plus grand dans la zone des deux arêtes longitudinales (2c, 2d) et le plus petit dans la zone de l'axe longitudinal médian (M).

7. Manchon de garrottage selon l'une quelconque des revendications précédentes, **caractérisé en ce que** la paroi expansible (4) est incurvée de manière convexe par leur expansion moyennant une déformation par cintrage de forme annulaire du support (3) et présente, dans l'état de base sans pression dans la zone de l'axe longitudinal médian (M) par rapport au support (3) une distance maximale (h), qui se situe dans la plage allant de 1 à 3 cm et de manière préférée est de l'ordre de 2 cm.

8. Manchon de garrottage selon l'une quelconque des revendications précédentes, **caractérisé en ce que** la chambre gonflable (1) s'étend entre une première section et une deuxième section du corps gonflable (2), dans lequel la première section (1a) est formée par la paroi expansible (4) et la deuxième section (1b) est formée par le support (3).

9. Manchon de garrottage selon l'une quelconque des revendications précédentes, **caractérisé en ce que** le matériau expansible est choisi parmi le groupe, lequel comprend des polymères élastomères, en particulier des élastomères thermoplastiques (TPE) et des élastomères terpolymères, en particulier du caoutchouc, du caoutchouc d'éthylène-propylène-diène (EPDM) et du caoutchouc d'éthylène-propylène (EPM), de la gomme et des silicones, ainsi que des tissus ou tricots expansibles, qui sont revêtus d'un matériau étanche à l'air et expansible.

10. Manchon de garrottage selon l'une quelconque des revendications précédentes, **caractérisé en ce que** le matériau expansible présente une dureté Shore dans la plage de 17 à 100 Shore A et/ou un module d'élasticité de 0,05 à 10 MPa.

11. Manchon de garrottage selon l'une quelconque des revendications précédentes, **caractérisé en ce que** la paroi expansible (4) est bombée de manière convexe dans la section transversale à la fois dans l'état de base sans pression et dans l'état gonflé du corps gonflable.

12. Manchon de garrottage selon l'une quelconque des revendications précédentes, **caractérisé en ce que** le corps gonflable (2) présente au moins une ouverture, laquelle peut être reliée à un conduit (15) pour relier le corps gonflable (2) à une source de gaz sous pression, dans lequel l'ouverture est disposée de préférence dans la zone d'une arête longitudinale (2c, 2d) ou d'une arête côté transversal du corps gonflable (2).

13. Manchon de garrottage selon l'une quelconque des revendications précédentes, **caractérisé en ce que** le support (3) est produit à partir d'une plaque en matière synthétique plane, en particulier à partir d'une matière synthétique thermoplastique, ou d'une tôle de métal.

14. Manchon de garrottage selon l'une quelconque des revendications précédentes, **caractérisé en ce que** le corps gonflable (2) est de forme stable déjà dans l'état de base sans pression et est sans pli et est cintré en forme toroïdale du fait de la précontrainte de la paroi expansible (4) sur le support (3), dans lequel le support (3) se situe radialement à l'extérieur et est cintré en forme annulaire du fait d'une tension de traction produite par la paroi expansible (4) étirée et les deux extrémités (2a, 2b) se faisant face du corps gonflable (2) se chevauchent ou reposent l'une au niveau de l'autre en forme d'aboutement.

15. Procédé servant à fabriquer un manchon de garrottage, lequel contient un corps gonflable (2), qui est formé à partir d'un support (3) allongé, s'étendant le long d'un sens longitudinal, composé d'un matériau de support étanche aux gaz et pouvant être cintré et d'une paroi expansible (4) fixée sur le support (3), composée d'un matériau expansible étanche aux gaz, dans lequel le procédé comprend des étapes suivantes :
- de fourniture d'un support (3) allongé s'étendant le long du sens longitudinal (L) avec une longueur de support (t) prédéfinie,
- de fourniture d'une paroi expansible (4) allongée s'étendant le long d'un sens longitudinal (L) avec une longueur (d) spécifiée, dans lequel la longueur (d) de la paroi expansible (4) est plus courte que la longueur de support (t) et la paroi expansible (4) présente des sections de bord (4a, 4b, 4c, 4d) périphériques,
- de fixation des sections de bord (4a, 4b, 4c, 4d) de la paroi expansible (4) en étirant le matériau expansible au moins dans le sens longitudinal (L) sur le support (3) allongé, de sorte qu'une chambre gonflable (1) étanche aux gaz se forme entre le support et la paroi expansible.
